# EUROPÄISCHE PATENTANMELDUNG

(11) **EP 3 333 249 A1**
(43) Veröffentlichungstag der Anmeldung: **13.06.2018**
(21) Anmeldenummer: 17184610.8
(22) Anmeldetag: 03.08.2017
(51) Int. Cl.: C12M 1/02, C12M 1/34, C12M 1/00, C10B 53/02

(54) **VERFAHREN UND VORRICHTUNG ZUR KURZWEGVERFLÜSSIGUNG VON BIOMASSE UND ÄHNLICHEN ORGANISCHEN STOFFEN (KOHLENWASSERSTOFF-VERBINDUNGEN)**

(30) Priorität: 03.08.2016 DE 102016114361
(71) Anmelder: Rosenkranz, Manfred, 78479 Reichenau (DE)
(72) Erfinder: Rosenkranz, Manfred, 78479 Reichenau (DE)
(74) Vertreter: Patentanwälte und Rechtsanwalt Weiß, Arat & Partner mbB

(57) **Zusammenfassung**

Eine Vorrichtung zur Verflüssigung von Biomasse aus den Bestandteilen Lignocellulose und Reststoffen auf der Basis von Kohlenwasserstoff, zur Erzeugung von Erdöl sowie Erdgas ähnlichen Produkten, die aus Öffnungen (E) und (F) abströmen, die Vorrichtung umfassend einen Reaktionsbehälter, soll dadurch gekennzeichnet sein, dass ein Ausgangsstoff durch eine Eingangsöffnung (2), durch welche ein Ausgangsstoff in die Reaktionskammer (1) eintreten kann sowie, dass
eine Düsenkombination (6), durch welche ein Oxidationsmittel, beispielsweise Sauerstoff, in den Reaktionsbehälter (1) einströmen kann.

## Beschreibung

### Technisches Gebiet

Die Erfindung betrifft ein Verfahren und eine Vorrichtung zur Kurzwegverflüssigung von Biomasse und ähnlichen organischen Stoffen (Kohlenwasserstoff-Verbindungen) mit dem Nebenprodukt eines brennbaren Synthesegases.

### Stand der Technik

Die enormen Mengen von fossilen Energieträgern, die aus der Förderung von Erdgas, Erdöl und Kohle stammen, werden der Menschheit in stofflicher und energetischer Form zur Verfügung gestellt. Das bei der Umwandlung entstandene CO₂ wird zum großen Teil durch Photosynthese in Biomasse umgewandelt. Wissenschaftliche Erhebungen beziffern die quantitative Bestimmung des gespeicherten Kohlenstoffs in Landökosysteme mit 2.800 Milliarden Tonnen.

Weltweit werden nach Alternativen und Verbesserungen zur Energie- und Rohstoffgewinnung gesucht.

Im Allgemeinen sind Derzeit Stoffumwandlungen, wie Kompostierung und energetische Nutzung, wie alkoholische Gärung und Gas aus Faultürmen bzw. Fermentationsanlagen im Einsatz.

Zu den heute bekannten Thermochemischen Umwandlungsverfahren zählen:
*1. Die Verbrennung*
   - Festbrennstoff, z.B. Holz, wird bei ca. 100 °C getrocknet;
   - Die Pyrolyse findet bei ca. 150 - 600 °C statt;
   - Die Oxidation läuft bei einer Temperatur von 900 - 1300 °C;
   - Dabei entsteht ein Rauchgas mit einer Temperatur von 600 - 700 °C.
*2. Vergasung*
   - Festbrennstoff, z.B. Holz, wird bei ca. 100 °C getrocknet
   - Die Pyrolyse findet bei ca. 150 - 600 °C statt;
   - Die Oxidation bzw. Reduktion liegt in einem Temperaturbereich zwischen 750 - 1000 °C
   - Dabei entsteht ein Schwachgas
*3. Langsame Pyrolyse*
   - Festbrennstoff, z.B. Holz, wird bei ca. 100 °C getrocknet;
   - Die Thermische Zersetzung findet bei einer Temperatur von 300 - 600 °C statt;
   - Dabei werden die folgenden Produkte erzeugt: ca. 80% Pyrolysegas; ca. 0-5% Pyrolyse-Öl; ca. 15% Pyrolysekoks
*4. Flash-Pyrolyse*
   - Festbrennstoff, z.B. Holz, bei ca. 475 °C findet eine Entgasung statt;
   - Durch die schnelle Gasabkühlung wird das Produkt verflüssigt
   - Dabei entstehen die folgenden Produkte: ca. 15 - 20% Pyrolysegas; ca. 65 - 75% Pyrolyse-Öl; ca. 10 - 15% Pyrolysekoks

Allgemein bezeichnet die Pyrolyse die Zersetzung von chemischen Produkten. Der Unterschied zu der Verbrennung oder Vergasung von Stoffen ist der, dass bei der Pyrolyse die thermochemische Spaltung unter Sauerstoffausschluss oder zumindest unterstöchiometrisch, stattfindet. Wodurch die Kohlenstoffverbindungen und die Kohlenwasserstoffverbindungen erhalten bleiben.

### Aufgabe der Erfindung

Aus dem Stand der Technik ist festzustellen, dass die aus Biomasse hergestellten Energie- und Rohstoffträger in Bezug auf Stoff- und Energiebilanz verbesserungswürdiges Potential in sich tragen.

Die vorliegende Erfindung hat es sich insbesondere zum Ziel gesetzt, bekannte Verfahren und Vorrichtungen zur Verflüssigung von Biomasse effizienter zu gestalten und zu verbessern. Unter Verflüssigung ist hierbei die Herstellung von Pyrolyseöl und Pyrolysegas zu verstehen.

### Lösung der Aufgabe

Gemäss der vorliegenden Erfindung wird neben Pyrolysegas bzw. Pyrolysekoks erstrangig und mehrheitlich Pyrolyseöl erzeugt. Vorzugsweise erfolgt diese Erzeugung dezentral und kontinuierlich und dadurch sehr zuverlässig. Es wird damit die technische Grundlage geschaffen, nicht nur elektrische Stromerzeugungsanlagen zu betreiben, sondern Biomasse mehrheitlich in einen Erdöl ähnlichen chemischen Grundstoff zu konvertieren.

Die thermochemischen und physikalischen Parameter: Druck, Temperatur, Zeit, Durchflussmenge sowie alle weiteren Bedingungen werden variabel gestaltet und an die Prozessbedingungen angepasst. Die Ausgestaltung der erfindungsgemässen Vorrichtung ist den äußeren klimatischen Gegebenheiten anzupassen.

Insbesondere ist zu beachten, dass unerwünschte Reaktionen, Produkte und Probleme vermieden werden. Dieses betrifft vor allem die Bildung von Pyrolysekoks. Während Pyrolysegas und Pyrolyseöl Energieträger sind, welche in vorteilhafter Weise weiter verarbeitet und energetisch genutzt werden können, muss der als Feststoff vorliegende Pyrolysekoks zumeist aufwändig entsorgt werden.

Zum Erreichen einer Betriebstemperatur ist ein energiereiches Medium einzusetzen, z.B. Propangas und von außen zu zuführen.

Die Betriebsbedingungen für die chemische Reaktion sind so ausgelegt, dass sie einem spontanen Lastwechsel standhalten.

Die Anlagentechnik ist durch kompakte Modulbauweise so ausgelegt und technisch bestimmt, dass bei einem Minimum an Aufwand für Produktion, Logistik sowie Lagerung, kohlenwasserstoffhaltige Rohstoffe zur Verfügung gestellt werden.
Die erfindungsgemässe Vorrichtung zur Verflüssigung von Biomasse aus den Bestandteilen Lignocellulose und Reststoffen auf der Basis von Kohlenwasserstoff, zur Erzeugung von Erdöl sowie Erdgas ähnlichen Produkten, die aus Öffnungen abströmen umfasst einen Reaktionsbehälter sowie eine Eingangsöffnung, durch welche ein Ausgangsstoff in die Reaktionskammer eintreten kann. Zuzüglich kann die Vorrichtung eine Düsenkombination enthalten, wodurch ein Oxidationsmittel, beispielsweise Sauerstoff, in den Reaktionsbehälter einströmen kann.

In der Vorrichtung kann ein energiehaltiges Öl sowie energiereiches Gas entstehen.

Der Ausgangsstoff kann durch eine Förderschnecke in den Reaktionsraum eintreten und sich vorteilsweise durch eine Trenn- und Leiteinrichtung, beispielsweise ein Prall- und Leitblech, verteilen. Durch Überwachung des Füllstandes ist ein Kontrollmechanismus über die Quantität gegeben.

Es kann daran gedacht sein, dass der Ausgangsstoff durch das Einströmen eines Oxidationsmittels über mehrere Düsen auf den Reaktionspartner trifft und keine weiteren Zusatzstoffe benötigt.

Es kann ferner daran gedacht sein, folgende Abschnitte in der Vorrichtung vorzusehen: Trocknung; Pyrolyse; Oxidation; Reduktion und Synthese; welche im Reaktionsbehälter stattfinden.

Es kann daran gedacht sein, dass über eine Zufuhr von Luftsauerstoff auf zumindest zwei Kammern eine Vorwärmung mit Verteilung des Oxidationsmittels für den Reaktionsprozess stattfindet.

Es kann ferner an eine Trennkammer gedacht sein, in welcher Energieparameter während des Reaktionsverlaufs auf Niveau gehalten werden.

In der Trennkammer kann eine Umströmung des Reaktionsbehälters durch Leiteinrichtungen gewährleistet werden

Es kann daran gedacht sein, dass in einem Ausgang des Reaktionsbehälters ein teilweiser Phasenübergang des Öl-Nebel-Gasgemisches in Kondensat, der Prozess der Verflüssigung fortgeführt wird.

Es kann ferner daran gedacht sein, dass in Strömungsrichtung mindestens eine Öffnung in die Öl/ Gas-Trenneinrichtung hinein ragt.

Ferner kann ein bestimmter Flächenabschnitt die Trenneinrichtung mit Öl-Nebel-Gas-Gemisch umströmen.

Es kann ein Energieaustausch zwischen der Öl/Gas-Trenneinrichtung und dem Wärmetauscher durch ein Medium stattfinden.

Das Pyrolyse-Öl kann in den Separationsbehälter abfließen und wird durch ein räumlich getrenntes Medium in seiner Viskosität konstant gehalten.

Es kann an ein Ventil mit Fluid-Verteiler in Strömungsrichtung gedacht sein, welches die Versorgung von nachgeschalteten thermo-chemischen - und physikalischen Aggregaten und Apparaturen übernimmt.

Ferner ist denkbar, dass das gereinigte Gas in Fließrichtung ein BHKW und/ oder einen Synthesereaktor versorgt.

Am Ausgang des Öl-Gas-Nebel-Gemisches kann ein Reinigungselement vorhanden sein.

Es kann an eine Zündeinrichtung gedacht sein, über welche unter Mitwirkung der Trennkammer eine notwendige Reaktionsenergie zur Verflüssigung der Biomasse erzeugt, um eine Oxidation des Ausgangsstoffes zu bewirken.

Die vorliegende Erfindung umfasst ferner ein Verfahren zur Verflüssigung von Biomasse aus den Bestandteilen Lignocellulose und Reststoffen auf der Basis von Kohlenwasserstoff, zur Erzeugung von Erdöl sowie Erdgas ähnlichen Produkten, die aus Öffnungen abströmen, wobei ein Ausgangsstoff durch eine Eingangsöffnung in eine Reaktionskammer eintritt.

Die Öffnungen können hierbei in einer mit dem Reaktionsbehälter fluidisch in Verbindung stehenden Öl/Gas-Trenneinrichtung vorgesehen sein.

Es ist denkbar, dass über eine Düsenkombination ein Oxidationsmittel, beispielsweise Sauerstoff in den Reaktionsbehälter strömt.

Ferner ist denkbar, dass der Reaktionsbehälter über die Zündeinrichtung unter Mitwirkung der Trennkammer die notwendige Initialenergie für die Reaktionsenergie erhält, um eine Oxidation des Ausgangsstoffes zu bewirken.

Ferner kann daran gedacht sein, nach dem Start der Reaktion keine weitere Initialenergie mehr zuzuführen.

Ferner kann daran gedacht sein, dass durch Hydrierung, Dampfreformierung und Elektrolyse die Energiekapazität vom Pyrolyseöl und Pyrolysegas wesentlich erhöht wird.

### Figurenbeschreibung

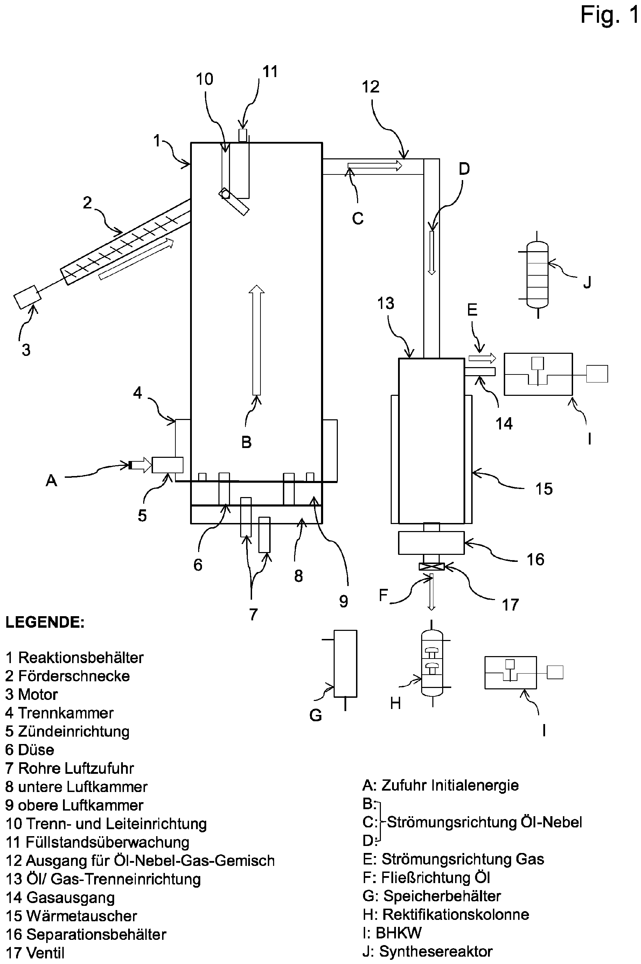

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in der Figur eine Vorrichtung gemäss einem Ausführungsbeispiel der vorliegenden Erfindung.

Die Grundstoffe Pyrolyse-Öl und Pyrolysegas stellen in allen industriellen Bereichen auf Grund der vorhandenen Infrastruktur Substitutionsstoffe dar.

Bei vorhandener Biomasse sind verkehrstechnisch aufwendig zu versorgende Gebiete mit entsprechender Anlagentechnik auszurüsten. Hierzu zählen Industrieunternehmen, urbane Gebiete sowie der gesamte ländliche Raum. Damit ist ein dezentraler Anschluss an moderne, gesellschaftliche Strukturen in Bezug auf Energie und Rohstoffe gegeben.

Ein Reaktionsbehälter (1) ist gleichermaßen als Trocknungsraum, Speicher und Reaktionskammer zu verstehen. Er wird mit einer Füllstandsüberwachung (11) ausgerüstet.

Eine Einbringung der Biomasse erfolgt mit Hilfe einer Förderschnecke (2). Diese bringt das Material, auch Inputmaterial oder Ausgangsstoff genannt, von der Seite in den Reaktionsapparat (1) ein.

Die aufwärts strömenden Gase (B) im Reaktionsraum (1) verlaufen im Gegenstromprinzip zum Inputmaterial und sind durch entsprechende Einrichtungen, wie beispielsweise ein Prall- und Leitblech (10), voneinander zu trennen.

Der eingebrachte Ausgangsstoff fließt gegen das Prall- und Leitblech (10) in den Reaktionsraum (1). Dort trocknet die auf einer Pyrolyseschicht aufliegende organische Masse des Inputmaterials weiter und geht in den Zustand der Pyrolyse über. Hierbei wird das Zellmaterial gecrackt und durch den Gasstrom aufwärts getragen. Es entstehen aus Zellulose in Verbindung mit Lignin feinste Molekularstrukturen.

Für den Reaktionsablauf ist der Ausgangsstoff in einer gleichmäßig definierten Korngröße, (z.B. genormte Holzpellets), mit vorgewärmtem Luftsauerstoff zu umspülen.

Durch die entsprechende Anordnung und Anzahl von Luftdüsen (6) wird das Inputmaterial mit dem Luftsauerstoff vermengt und zur Reaktion gebracht.

Die Luftdüsen (6) halten die Temperatur in der Reaktionskammer (1) zwischen 250 bis 350 °C relativ konstant. Dadurch verläuft die Entstehung des Pyrolyse-Öls weitestgehend schonend.

Die Düsenkonfiguration verbindet die Prinzipien aus Wirbelschicht- und Festbett-Vergasung und wird in Luftkammern (8, 9) vorbereitet.

Beim Startvorgang wird eine Trennkammer (4) mit Initialenergie gefüllt und löst unter Zugabe von Luftsauerstoff den Vergasungsprozess im Reaktionsraum (1) aus.

Die Austrittsöffnung (12) des Reaktionsbehälters bewirkt ein Abströmen (Pfeil C) des Öl-Gas-Gemisches zu einer mit dem Reaktionsbehälter fluidisch verbundenen Kühleinrichtung bzw. Öl/Gas-Trenneinrichtung (13) mit Übergang zum Separations-Behälter (16). Die Temperatur des Öles und des Gases wird in Fließrichtung (Pfeil D) nach dem Gegenstromprinzip durch Wärmetauscher (15) schlagartig abgekühlt und lassen das Pyrolyse-Öl entstehen.

Während des kontrollierten, geregelten und überwachten Prozesses reagieren der Kohlenstoff (C) und der Sauerstoff (O₂) exotherm und es entsteht zunächst Kohlenstoffdioxid (CO₂). Durch Temperaturanhebung spaltet sich das CO₂ in Reaktion mit C zu Kohlenstoffmonoxid (2 x CO). Ebenso wird eine Vielzahl von leichten und schweren Kohlenwasserstoffverbindungen gebildet wie beispielsweise Methan (CH₄). Ferner wird Wasserstoff (H₂) gebildet.

Eine auf den Ablauf abgestimmte Trenntechnik bzw. Öl/Gas-Trenneinrichtung (13) entnimmt die feinen Öltropfen aus dem Öl-Nebel-Kondensat.

In einem Behälter (16) wird das Öl aufgefangen und periodisch entnommen. Ein Fluid mit hoher Wärmekapazität hält den Prozessablauf stabil.

Der Massenanteil des Pyrolyse-Öles beträgt ca. 65 bis 70% vom Ausgangsstoff, das Pyrolysegas hat einen Massenanteil von ca. 30 bis 35%. Es entsteht ca. 0 bis 1% Pyrolysekoks.

Der Reaktionsbehälter (1) ist mit einer auf mehreren Ebenen abgestimmten Trenn- und Leiteinrichtung, beispielsweise einer Trennkammer (4), umgeben. Diese thermische Einrichtung (4) vollzieht einen ständigen Wärmeaustausch und hält die Prozessenergie konstant.

Während des Prozesses wird kontinuierlich Input-Materialnachgefördert, beispielsweise mit Hilfe der Förderschnecke (2). Der Prozess verläuft in einem gut beherrschbaren und sehr robusten Umfeld.

Mit der Lösung aller Aufgaben wird der Anspruch auf eine regenerative Rohstoffkonvertierung in den Punkten: hoher Wirkungsgrad, Speicherbarkeit sowie Transportwürdigkeit erfüllt.

## Patentansprüche

1. Vorrichtung zur Verflüssigung von Biomasse aus den Bestandteilen Lignocellulose und Reststoffen auf der Basis von Kohlenwasserstoff, zur Erzeugung von Erdöl sowie Erdgas ähnlichen Produkten, die aus Öffnungen (E) und (F) abströmen,
die Vorrichtung umfassend einen Reaktionsbehälter (1),
**gekennzeichnet durch**
eine Eingangsöffnung (2), durch welche ein Ausgangsstoff in die Reaktionskammer (1) eintreten kann sowie
eine Düsenkombination (6), durch welche ein Oxidationsmittel, beispielsweise Sauerstoff, in den Reaktionsbehälter (1) einströmen kann.

2. Vorrichtung nach Anspruch 01, **dadurch gekennzeichnet, dass** ein energiehaltiges Öl sowie energiereiches Gas entsteht.

3. Vorrichtung nach Anspruch 01 und 02, **dadurch gekennzeichnet, dass** der Ausgangsstoff durch die Förderschnecke (2) in den Reaktionsraum (1) eintritt und sich vorteilsweise durch die Trenn- und Leiteinrichtung (10) verteilt.

4. Vorrichtung nach Anspruch 02, **dadurch gekennzeichnet, dass** der Ausgangsstoff durch das Einströmen eines Oxidationsmittels über mehrere Düsen (6) auf den Reaktionspartner trifft und keine weiteren Zusatzstoffe benötigt.

5. Vorrichtung nach Anspruch 02 und 03, **dadurch gekennzeichnet, dass** die Abschnitte: Trocknung; Pyrolyse; Oxidation; Reduktion und Synthese, im Reaktionsbehälter (1) stattfinden.

6. Vorrichtung nach Anspruch 03 und 04, **dadurch gekennzeichnet, dass** über die Zufuhr von Luftsauerstoff (7) auf die Kammern (8) und (9) eine Vorwärmung mit Verteilung des Oxidationsmittels für den Reaktionsprozess stattfindet.

7. Vorrichtung nach Anspruch 03; 04 und 05, **dadurch gekennzeichnet, dass** in der Trennkammer (4) die Energieparameter während des Reaktionsverlaufes auf Niveau gehalten werden.

8. Vorrichtung nach Anspruch 03; 04 und 05, **dadurch gekennzeichnet, dass** in der Trennkammer (4) die Umströmung des Reaktionsbehälters (1) durch Leiteinrichtungen gewährleistet ist.

9. Vorrichtung nach Anspruch 01 und 02, **dadurch gekennzeichnet, dass** im Ausgang (12) den teilweisen Phasenübergang des Öl-Nebel-Gemisches in Kondensat, der Prozess der Verflüssigung fortgeführt wird.

10. Vorrichtung nach Anspruch 01 und 02, **dadurch gekennzeichnet, dass** in Strömungsrichtung (D) mindestens eine Öffnung in die Öl/ Gas-Trenneinrichtung (13) hinein ragt.

11. Vorrichtung nach Anspruch 01 und 02, **dadurch gekennzeichnet, dass** ein bestimmter Flächenabschnitt die Trenneinrichtung (13) mit Öl-Nebel-Gas-Gemisch umströmt.

12. Vorrichtung nach Anspruch 01 und 02, **dadurch gekennzeichnet, dass** ein Energieaustausch zwischen der Öl/Gas-Trenneinrichtung (13) und dem Wärmetauscher (15) durch ein Medium stattfindet.

13. Vorrichtung nach Anspruch 01; 02; 10; 11, **dadurch gekennzeichnet, dass** das Pyrolyse-Öl in den Separationsbehälter (16) abfließt und durch ein räumlich getrenntes Medium in seiner Viskosität konstant gehalten wird.

14. Vorrichtung nach Anspruch 01; 02; 10; 11; 12, **dadurch gekennzeichnet, dass** ein Ventil (17) mit Fluid-Verteiler in Strömungsrichtung (F) die Versorgung von nachgeschalteten thermochemischen -und physikalischen Aggregaten und Apparaturen (G: Speicherbehälter; H: Rektifikationskolonnen; I: BHKW) übernimmt.

15. Vorrichtung nach Anspruch 01; 02; 10; 11; 12, **dadurch gekennzeichnet, dass** das gereinigte Gas in Fließrichtung (E) ein BHKW (I) und/ oder einen Synthesereaktor (J) versorgt.

16. Vorrichtung nach Anspruch 01 und 02, **dadurch gekennzeichnet, dass** am Ausgang des Öl-Gas-Nebel-Gemisches (12) ein Reinigungselement vorhanden ist.

17. Vorrichtung nach zumindest einem der vorhergehenden Ansprüche, **gekennzeichnet durch** eine Zündeinrichtung (5), über welche unter Mitwirkung der Trennkammer (4) eine notwendige Reaktionsenergie zur Verflüssigung der Biomasse erzeugt wird, um eine Oxidation des Ausgangsstoffes zu bewirken.

18. Verfahren zur Verflüssigung von Biomasse aus den Bestandteilen Lignocellulose und Reststoffen auf der Basis von Kohlenwasserstoff, zur Erzeugung von Erdöl sowie Erdgas ähnlichen Produkten, die aus Öffnungen (E) und (F) abströmen,
**dadurch gekennzeichnet, dass**
ein Ausgangsstoff durch eine Eingangsöffnung (2) in eine Reaktionskammer (1) eintritt.

19. Verfahren nach Anspruch 18, **dadurch gekennzeichnet, dass** über eine Düsenkombination (6) ein Oxidationsmittel (O₂) in den Reaktionsbehälter (1) strömt.

20. Verfahren nach einem der Ansprüche 18 oder 19, **dadurch gekennzeichnet, dass** der Reaktionsbehälter (1) über die Zündeinrichtung (5) unter Mitwirkung der Trennkammer (4) die notwendige Initialenergie in Form von Reaktionsenergie erhält, um eine Oxidation des Ausgangsstoffes zu bewirken.

21. Verfahren nach einem der Ansprüche 18 bis 20, **dadurch gekennzeichnet, dass** nach einem Start der Reaktion keine weitere Initialenergie zugeführt wird.

22. Verfahren nach einem der Ansprüche 18 bis 20, **dadurch gekennzeichnet, dass** durch Hydrierung, Dampfreformierung und Elektrolyse die Energiekapazität vom Pyrolyseöl und Pyrolysegas wesentliche erhöht wird.
